# EUROPEAN PATENT SPECIFICATION

(11) **EP 3 123 975 B1**
(45) Date of publication and mention of the grant of the patent: **13.02.2019**
(21) Application number: 16181809.1
(22) Date of filing: 28.07.2016
(51) Int. Cl.: A61C 9/00, A61C 8/00

(54) **SCANBODY**
SCANKÖRPER
CORPS DE SCANNER

(30) Priority: 31.07.2015 BR 102015018460
(43) Date of publication of application: 01.02.2017
(73) Proprietor: JJGC Indústria e Comércio de Materiais Dentários S.A., 81270-200 Curitiba (Parana) (BR)
(72) Inventor: THOMÉ, GENINHO, 80270-200 Curitiba (Paraná) (BR); THOMÉ, THIAGO VIEIRA, 80270-200 Curitiba (Paraná) (BR); MERTIN, FELIX ANDREAS, 80270-200 Curitiba (Paraná) (BR)
(74) Representative: Elzaburu S.L.P.

(56) References cited:
- EP-A2- 2 457 536
- KR-B1- 101 452 849

## Description

### Scope

The present invention relates to the field of dentistry and, specifically, to printing aided techniques used for dentures and molds for printing. This invention relates to a component adapted to transfer position, direction and rotation information of an implant installed in a patient's mouth or in a mold to be used in scanning equipment and the manufacturing of customized prostheses.

### Technical status

Dental implants, widely known in the technique, are used as supports to replace one or more missing teeth. The implant, also known as abutment, is the first of at least two parts of dental restoration that also includes one or more prosthetic elements fixed to the implant by means of a screw. The prosthetic elements are responsible for aesthetic replacement of missing teeth and usually consist of an intermediate structural component known as connection; and aesthetic replacement, known as crown, commonly attached through adhesive or cement suitable for dental use. In some cases only a prosthetic component is used, combining structural and aesthetic function, for one or more missing teeth.

The success of restoration depends on the stability of the entire set, particularly the absence of relative movement between the abutment and the prosthetic elements. For this purpose, abutment typically includes, in its upper portion, a hexagonal or octagonal-shaped coupling as anti-rotation element, or other geometric form to prevent rotation, even amorphous, which is applied directly to surface or inside a conical recess usually known as Morse taper.

The prosthetic element, in turn, is coupled to abutment, topically connection, and includes complementary coupling geometry, whether anti-rotation or not (as the case may be), so that when connection and abutment are coupled in the mouth, the cooperation between anti-rotation elements prevents the relative movement between these components or, in case of prosthesis supported on multiple implants, the disposition itself of more than one supporting point to prevent said relative movement. Thus, the screw that holds the connection to abutment is used just to prevent the separation of these two parts and do not prevent the relative rotation movement among them, by minimizing the strain on the screw and expanding its cycle life.

The presence of these anti-rotation elements requires additional care on the manufacture of prosthetic component so that, when installed, the internal surfaces and external prosthesis remain aligned to those of nearby teeth. For this purpose, it is necessary to measure not only the position (x, y, and z), but also the direction (in angles around each one of the axes x, y, and z), as well as the anti-rotation fixing element alignment in the mouth, that is, in which direction the corners of this anti-rotation element are located, so that these information are considered in the manufacture of the connection and reflected on how the aesthetic portion is concocted in relation to underlying anti-rotation element. This occurs because, even if the installation is carefully made to achieve a specific alignment, certain micro movements during the healing phase may lead to loss of alignment.

When the prosthetic element is supported on multiple implants, for instance, in case of a prosthetic bridge, the position and direction of the abutment is vital information to avoid prosthesis settlement issues that may occur when, due to misalignment of given connection elements makes up the bridge, the dentist is bound to force or fit the component so that it can be implanted.

Currently, after a period of healing in which the implant remains closed under the gums, it is exposed in the mouth for the mounting of a healing connection. During the process, the position of the implant can be captured by physical molding or directly to the computer by means of scanning process. During the physical molding process, a printing workpiece (as described in document US5829981) capable of connecting the anti-rotation element of the implant is stuck to it and the printing of the whole mandibular arch is made by capturing such workpiece. Afterwards, the printing is removed from mouth and a component similar to the implant is fixed to the same workpiece and the set gets a coat of plaster or a similar molding material so as to get a model of the patient's mandibular arch after the healing of the implant.

Formerly, this step was followed by manual molding of the desired prosthetic component, generally in wax, for further casting in biocompatible metal (E.g.: gold) and aligning problems were solve by adding intermediary components as those described in patent US4988297. However, CAD/CAM technologies for prosthetic components, as described in document US4742464 and, particularly, from blocks that include the prefabricated anti-rotation element, as shown in patent US6991853, have paved the way for the manufacturing of connections through the computer without the need for manual molding or wax works. For this purpose, data about the position and orientation of the implant needs to be informed to the computer, which is done by means of intra-oral scanning (E.g.: laser infrared cameras or scanners) as described in patents US4575805 and US4837732.

However, it is difficult to work with scanning media directly inside the mouth, especially due to access difficulties, limited space, poor lighting, and the presence of fluids (like saliva), which cause undesired reflexes that may affect the quality of the scanning and compromise the accuracy of the measurement. Capturing the anti-rotation element at the top of the implant when it might be hidden, in fluids or covered by close elements is particularly challenging, which frequently leads to errors that need to be later balanced.

In order to prevent errors, CAD/CAM system manufacturers started to employ measurement devices or scanning transfers, as indicated by reference number 35 on US8480396, which is intended to highlight the position and orientation of the implant for the scanning device. Such transfers may be placed in the mouth or over the plaster mold produced with the help of a printing piece, as previously performed. The advantage of the mold is the possibility of sending it to a laboratory when the dentist does not have the equipment for intra-oral scanning. The use of the mold as basis for the scanning of the implant's position and orientation allows the use of scanning methods that are cheaper and larger, which cannot be used intra-orally.

Scanbodies used to determine the position and orientation of components as implants are endowed with a well-known technique. For instance, document US8747112 describes a workpiece to be fit in a dental implant in an orientation that defines an 'X' insertion central axis and a 'D' insertion direction of the workpiece. The workpiece includes a flat bevel angle in its upper portion and a fit connection, and the implant has a resilient spring member in its lower portion for preventing its displacement relative to the implant when inserted into the dental implant.

Also, EP2130514 describes a scanning item endowed with beveled surface to enable optical scanning. The flat bevel angle extends itself over the length of the workpiece and its larger part is next to the base and its smaller part is closer to the top, and the width between the two edges decreases steadily. The document also mentions that the member is used solely for detection, and the same may be done in low-reflection material, reducing the need for adding non-reflective material.

As per aforementioned documents as well as according to components available in the market, state-of-the-art scanbodies use flat surfaces over a generally cylindrical body to determine the orientation of the implant and, specifically, the orientation of the anti-rotation element of the implant. This flat surface is generally in the form of a laminate or key way or, as in the case presented in document EP2218423, a prismatic element comprised of multiple flat surfaces.

The problem with current scanners is that its geometry generate artifacts (also referred to as errors) during scanning. Such artifacts occur due to the way the light emitted by scanners interacts with flat surfaces of the pieces. Such errors are compensated in a post-processing step, which prolongs the scanning process. Sometimes, when the measuring error is significant even after further processing, it is necessary to cover the piece with opaque sprays or non-reflective powder before a new measurement is performed.

Furthermore, when the piece does not present flat surfaces, as in case of main achievement presented in document EP2400917, it is not possible to locate the orientation of the anti-rotation element on abutment head. The scanbody as per EP2400917 is also subject to problems in the identification of implant position and direction when it is set between remaining dental elements and only functions efficiently in case of tooth loss.

Also, the material currently used in the making of scanners presents problems. Metal used in several aforementioned manners is expensive and presents shiny surfaces after machining. The plastic materials, on the other hand, are manufactured by an injection process to minimize costs; however, the injection results in a nearly polished finishing, which highlights the shine problems, demanding the use of opaque sprays. Among the plastic materials used in the injection process, natural Polyether Ether Ketone (PEEK) is one of the most widely used; however, it does not present good performance during the scanning process due to reflection. A more efficient product is required, which can be totally and flawlessly scanned and which guarantees quality and accuracy to the scanning process in any type of scanner.

EP 2 457 536 A2 describes a device has a three-dimensional scanning area provide on the implant base portion for detecting the unambiguous surface of patient from various scanning parameters; and a transition region is formed between the implant base portion and scanning area. Several contour portions are distributed and arranged along the periphery of scanning area. The contour portions are projected radially towards the outer portion of transition region.

### Summary

The improvement in scanbody performance depends on two characteristics: raw material and external geometry. Regarding raw material, it is necessary to effect on opaque material that can be used in CAD/CAM scanners, which is used to dispense powder or spray application during scanning process. Regarding geometry is necessary to provide a geometry that favors the scanning in all the regions of the mouth with the correct identification of the position, direction and rotation of the implant or implant analogs. The scanbody should be asymmetrical to ensure the correct identification of the position, direction and rotation of the analogs, without generating any asymmetry measurement errors.

One of the objectives of the present invention is to provide a scanbody geometry for scanning purposes that minimizes or preferably eliminates the formation of artifacts (errors) during the scan, so that to increase the accuracy and reliability of the scanning of models with analogs, thus facilitating the manufacture of prosthetic structures. A second objective of the invention is to eliminate the need of using non-reflective powders or opacificator sprays during scanning process.

These objectives are achieved by assembling a scanbody that uses only rounded surfaces on the face to determine the rotational orientation of the workpiece and by applying dual-taper geometry design to scanbody. When replacing flat surfaces by rounded ones, the quality of data collection is improved, since such surfaces decrease the incidence of reflections on the scanner's capture element. In addition, to enlarge the reading area without generating unwanted reflections, dual-taper geometry that forms the scan body allows to determine workpiece height more precisely. The facility to recognize geometry affects conveniently the software needed for the scanning process, since less error correction routines are required during scanning.

Machining the part instead of manufacturing it by injection molding also contributes to achieve invention objectives since the micro grooves resulting from machining process helps increase scanbody surface opacity.

### Brief description of the drawings

The scanbody of the present invention is described below by referring to drawings, which:
Figures 1A and 1B illustrate the first variant and a cross-sectional view of the proposed scanbody.
Figures 2A and 2B illustrate a second variant endowed with hole for fixing screw access.
Figures 3, 4, 5 and 6 illustrate variants of this invention provided with different coupling elements. Figures 7A and 7B illustrate the effect achieved by the surfaces of the present invention during scanning operation.
Figures 8A and 8B illustrate, for comparison purposes, the previous technique issue during scanning procedure.

### Description

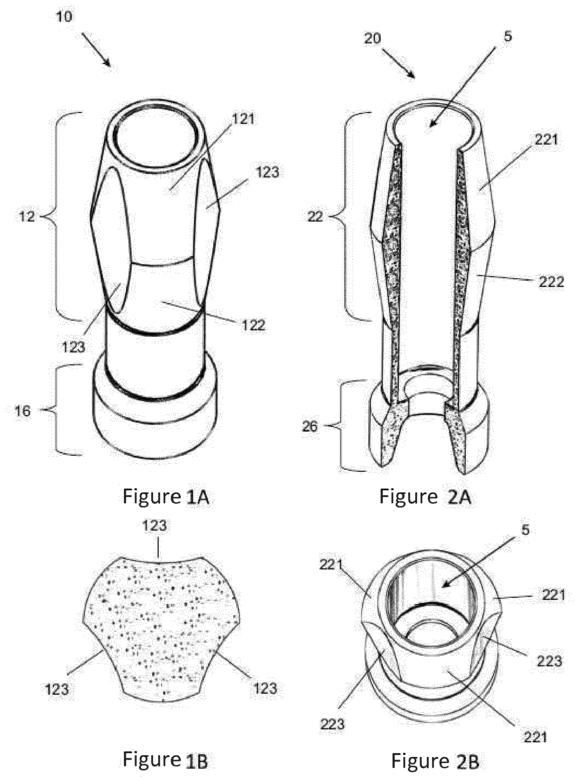

Figure 1 illustrates the main features of the proposed scanbody device. The scanning device (10) is comprised of a body (12) and a base (16) interconnected by a middle part to maintain body and base set height constant when different types of anti-rotation elements are used.

The base (16) lower part fits on the dental implant anti-rotation geometry, whether located in a fixing installed in the mouth or analog attached to a plaster model. As seen in related numbers in figures 2A, 3, 4, 5, and 6, different base geometries (16, 26, 36, 46, 56, 66) can be used, always relating to the type of abutment coupling geometry used as the case may be.

The body (12) is formed by a dual-taper geometry, specifically formed by two truncated cones joined base-to-base that will be called primary and secondary trunconical elements from now on. The main feature of the body that allows the invention to reach its objectives is the fact that its dual-trunconical assembly occurs without any flat surface.

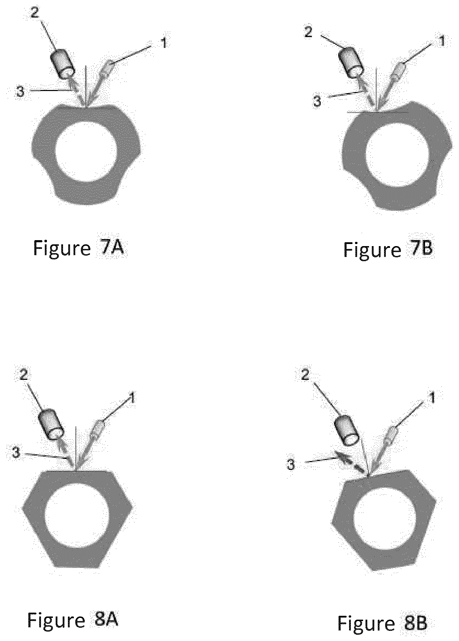

Rounded surfaces, as schematically seen in figures 7A and 7B, to be irradiated by a source of light (1) always feature at least one light reflection point that carries part of the light waves reflected (3) towards a receiver (2). The time range between light wave emission and detection is used to measure the distance traveled and, consequently, the geometry of the workpiece being scanned.

When a workpiece features flat surfaces, as shown in figures 8A and 8B, in some points no wave (3) irradiated by a source of light (1) reaches the receiver (2). This measurement fault results in geometry estimation errors if they are seen as irregular spots on the surface of the scanned part when displayed on computer. In extreme cases, such reading errors are enough to mask the exact geometry leading to manufacturing failed prosthetic element that need to be corrected after manufacture. The current technique corrects this type of problem with post processing steps inside the computer. Which requires more sophisticated computer programs and longer processing time. Thus, the present invention solves the same problem by changing the geometry of the scanbody.

It is noted that the different variants of the scandoby that is subject matter of the present invention, the body (12, 22, 32, 42, 52, 62) arranged for scanner reading always display the opposing primary trunconical element (121, 221, 321, 421, 521, 621), base-to-base to a secondary trunconical element (122, 222, 322, 422, 522, 622). This dual-trunconical geometry increases the reading area, and it is essential to determine implant position, particularly when installing the implant in the mouth. This greater reading area increases implant position transfer during scanning to minimize settlement failure problems of the final prosthesis.

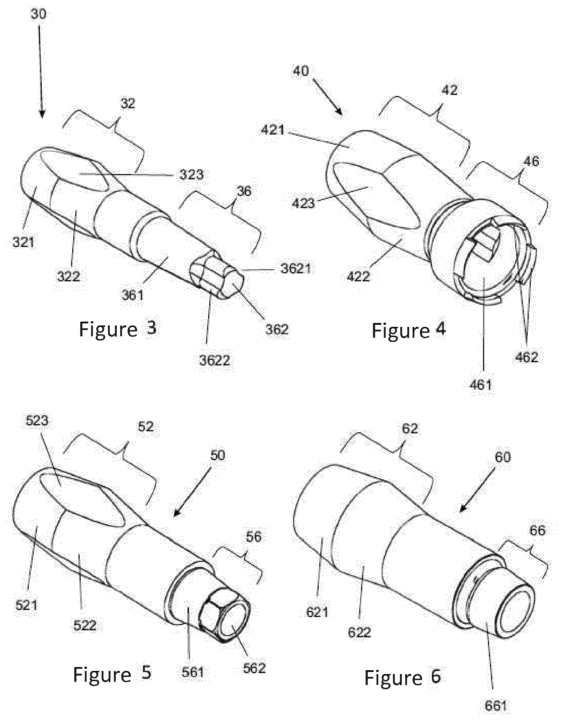

In some cases, for example, manufacture of bridges, which are prostheses supported on multiple implants in which the body, with two trunconical elements, is enough to detect implant position and direction information. This occurs because, in those cases, since it has multiple supporting points, the prosthetic element does not depend on the anti-rotation geometry found in the head of the implant to ensure its alignment within the mouth. Figure 6 illustrates the scan component to be used in such case.

In other cases, as in the example of a single tooth replacement, not only position and direction, but also the of the rotating location of the anti-rotation element needs to be determined, so that the prosthetic component is placed in the same position to be aligned with the rest of the dental arch. To comply with those cases, the invention can additionally comprise concave surfaces (123, 223, 323, 423, 523) arranged in the side of the body (12, 22, 32, 42, 52) so that allows measuring the rotational information without making use of flat surfaces.

Figures 2A and 2B illustrate a scanbody according with the present invention that additionally includes a hole (5) and channel that fits a fixing screw. Although optional, the use of fixing screws eases scanbody attachment to the implant, either in the patient mouth or in the model, avoiding displacements during scanning.

Figures 3, 4, 5 and 6 illustrates variants of this invention provided with different coupling elements in its base. Figures 3, 4 and 5 illustrate anti-rotation coupling geometries, while Figure 6 shows rotating coupling geometry, the last being used for prosthesis supported on multiple implants when the position of the anti-rotation implant element is not relevant.

Figure 3 illustrates the scanbody (30) with Morse taper (361) anti-rotation geometry on its base (36) associated to a prism (362) with three concave sides (3621) lodged within three convex sides (3622).

Figure 4 in its turn illustrates the scanbody (40) with Universal type (461) anti-rotation geometry at the base (46), that is, it can be coupled to different types of anti-rotation geometries associated with the scanbody fastening clamps (462) during scanning operation.

Figure 5 in its turn illustrates a scanbody (50) with Morse taper (561) type anti-rotation geometry on base (56) associated with an hexagonal prism (562). Figure 6 in its turn illustrates the scanbody (60) with straight trunconical (661) rotation coupling geometry at the base (66), which is useful in the event that multiple implants are used to support, for example, a prosthetic bridge, as previously mentioned, in case such rotating orientation information is not so relevant.

Different opaque materials may be used employed to produce scanbodies using he geometry of the present invention. Preferably, such materials should be biocompatible and be autoclaved for disinfection to minimize the occurrence of problems when the scanbodies are employed in intraoral environment. Laboratory assays showed that it can advantageously be applied to PEEK-ClassixTM white resin, developed by Invibio®, and life science grade by-products such as Ketron® PEEK LSG plastic, whose opacity characteristics are higher than those of natural PEEK. Also aiming at the improvement of the scanbody opacity characteristics for the present invention, it is machined instead of injection molding as scanbodies are made according to such technique. The micro surface resulting from the machining process brings more benefits in terms of lower reflectivity (greater opacity) of the piece.

## Claims

1. Scanbody (10, 20, 30, 40, 50, 60) comprising a base (16, 26, 36, 46, 56. 66) with coupling geometry which is adapted for fitting in an anti-rotation geometry of a dental implant and a body (12,22, 32, 42, 52, 62) prepared for scanning; wherein the lateral surface of the body has geometrical elements (121, 122, 221, 222, 321, 322, 421, 422, 521, 522, 621, 622) which allow the identification of information regarding the scanbody position, direction and rotation, wherein the lateral surface of the body does not display any flat areas, **characterized in that** the body (12,22, 32, 42, 52, 62) is formed by a dual-taper geometry formed by primary (121, 221, 321, 421, 521, 621) and secondary (122, 222, 322, 422, 522, 622) trunconical elements which are joined base-to-base.

2. Scanbody according to Claim 1, **characterized by** the fact that, a concave surface (12, 22, 32, 42, 52) is arranged at the side of the body (123, 223, 323, 423, 523).

3. Scanbody according to any of Claims 1 or 2, **characterized by** the fact that it includes a hole (5) and channel that fits a fixing screw.

4. Scanbody (3) according to any of Claims 1 to 3, **characterized by** the fact that it comprises a Morse taper (361) anti-rotation geometry on its base associated to a prims with three concave sides (3621) lodged within three convex sides.

5. Scanbody (40) according to any of Claims 1 to 3, **characterized by** the fact that the coupling geometry on the base (46) is of a universal type (461) associated with fastening clamps (362) to secure the scanbody.

6. Scanbody (50) according to any of Claims 1 to 3, **characterized by** the fact that the coupling geometry on the base (56) is of a morse taper type (561) associated with a hexagonal prism (362).

7. Scanbody (60) according to any of Claims 1 to 3, **characterized by** the fact that the coupling geometry on the base (66) is of a straight trunconic type (661).

8. Scanbody (10, 20, 30, 40, 50, 60), according to any of Claims 1 to 7, **characterized by** the fact that it is manufactured from biocompatible material.

9. Method of manufacturing a scanbody according to any of claims 1 to 8, **characterized by** the fact that the scanbody is machined with numerical control of tools.

## Patentansprüche

1. Scankörper (10, 20, 30, 40, 50, 60), umfassend eine Basis (16, 26, 36, 46, 56, 66) mit Kopplungsgeometrie, die angepasst ist, um in eine Verdrehsicherungsgeometrie eines Zahnimplantats und eines Körpers (12, 22, 32, 42, 52, 62) einzupassen, die zum Scannen vorbereitet ist; wobei die seitliche Fläche des Körpers geometrische Elemente (121, 122, 221, 222, 321, 322, 421, 422, 521, 522, 621, 622) aufweist, welche die Identifizierung von Informationen zur Position, Richtung und Rotation des Scankörpers ermöglichen, wobei die seitliche Fläche des Körpers keine ebenen Flächen zeigt, **dadurch gekennzeichnet, dass** der Körper (12, 22, 32, 42, 52, 62) von einer beidseitig spitz zulaufenden Geometrie gebildet ist, die von primären (121, 221, 321, 421, 521, 621) und sekundären (122, 222, 322, 422, 522, 622) kegelstumpfförmigen Elementen, die von Basis zu Basis verbunden sind, gebildet wird.

2. Scankörper nach Anspruch 1, **dadurch gekennzeichnet, dass** eine konkave Fläche (12, 22, 32, 42, 52) an der Seite des Körpers (123, 223, 323, 423, 523 angeordnet ist.

3. Scankörper nach einem der Ansprüche 1 oder 2, **dadurch gekennzeichnet, dass** er eine Öffnung (5) und einen Kanal enthält, der in eine Fixierschraube einpasst.

4. Scankörper (3) nach einem der Ansprüche 1 bis 3, **dadurch gekennzeichnet, dass** er eine Verdrehsicherungsgeometrie in Form eines Morsekegels (361) an seiner Basis umfasst, die mit einem Prisma mit drei konkaven Seiten (3621) in Verbindung ist, das innerhalb der drei konkaven Seiten aufgenommen ist.

5. Scankörper (40) nach einem der Ansprüche 1 bis 3, **dadurch gekennzeichnet, dass** die Kopplungsgeometrie an der Basis (46) von einem universellen Typ ist (461), in Verbindung mit Befestigungsklemmen (362), um den Scankörper zu sichern.

6. Scankörper (50) nach einem der Ansprüche 1 bis 3, **dadurch gekennzeichnet, dass** die Kopplungsgeometrie an der Basis (56) vom Typ spitz zulaufend (561) ist, in Verbindung mit einem hexagonalen Prisma (362).

7. Scankörper (60) nach einem der Ansprüche 1 bis 3, **dadurch gekennzeichnet, dass** die Kopplungsgeometrie an der Basis (66) vom Typ gerade kegelstumpfförmig (661) ist.

8. Scankörper (10, 20, 30, 40, 50, 60), nach einem der Ansprüche 1 bis 7, **dadurch gekennzeichnet, dass** er aus biologisch verträglichem Material hergestellt ist.

9. Verfahren zum Herstellen eines Scankörpers nach einem der Ansprüche 1 bis 8, **dadurch gekennzeichnet, dass** der Scankörper mit nummerischer Steuerung der Werkzeuge bearbeitet wird.

## Revendications

1. Corps de scanneur (10, 20, 30, 40, 50, 60) comprenant une base (16, 26, 36, 46, 56, 66) avec une géométrie de couplage qui est à même de s'ajuster dans une géométrie anti-rotation d'un implant dentaire et un corps (12, 22, 32, 42, 52, 62) préparé pour un balayage ; dans lequel la surface latérale du corps a des éléments géométriques (121, 122, 221, 222, 321, 322, 421, 422, 521, 522, 621, 622) qui permettent l'identification d'informations concernant la position, la direction et la rotation du corps de scanneur, dans lequel la surface latérale du corps n'affiche pas de zones plates quelconques, **caractérisé en ce que** le corps (12, 22, 32, 42, 52, 62) est formé par une géométrie à double conicité constituée par des éléments tronconiques primaires (121, 221, 321, 421, 521, 621) et secondaires (122, 222, 322, 422, 522, 622) qui sont joints base à base.

2. Corps de scanneur selon la revendication 1, **caractérisé en ce qu'**une surface concave (12, 22, 32, 42, 52) est agencée sur le côté du corps (123, 223, 323, 423, 523).

3. Corps de scanneur selon l'une quelconque des revendications 1 ou 2, **caractérisé en ce qu'**il comprend un trou (5) et un canal qui reçoivent une vis de fixation.

4. Corps de scanneur (3) selon l'une quelconque des revendications 1 à 3, **caractérisé en ce qu'**il comprend un géométrie anti-rotation à conicité Morse (361) sur sa base associée à un prisme ayant trois côtés concaves (3621) logés dans trois côtés convexes.

5. Corps de scanneur (40) selon l'une quelconque des revendications 1 à 3, **caractérisé en ce que** la géométrie de couplage sur la base (46) est d'un type universel (461) associé à des pinces de fixation (362) pour fixer le corps de scanneur.

6. Corps de scanneur (50) selon l'une quelconque des revendications 1 à 3, **caractérisé en ce que** la géométrie de couplage sur la base (56) est d'un type de conicité Morse (561) associé à prisme hexagonal (362).

7. Corps de scanneur (60) selon l'une quelconque des revendications 1 à 3, **caractérisé en ce que** la géométrie de couplage sur la base (66) est d'un type tronconique droit (661).

8. Corps de scanneur (10, 20, 30, 40, 50, 60) selon l'une quelconque des revendication 1 à 7, **caractérisé en ce qu'**il est fabriqué à partir d'un matériau biocompatible.

9. Procédé de fabrication d'un corps de scanneur selon l'une quelconque des revendication 1 à 8, **caractérisé en ce que** le corps de scanneur est usiné avec une commande numérique d'outils.
